# EUROPÄISCHE PATENTANMELDUNG

(11) **EP 1 369 411 A1**
(43) Veröffentlichungstag der Anmeldung: **10.12.2003**
(21) Anmeldenummer: 02291411.3
(22) Anmeldetag: 07.06.2002
(51) Int. Cl.: C07C 69/612, C07C 69/618, C12P 23/00, A61K 7/42

(54) **Kosmetische und/oder pharmazeutische Zubereitungen mit Retinolestern**

(71) Anmelder: Cognis France S.A., 31360 Saint-Martory (FR)
(72) Erfinder: Pauly, Gilles, 54000 Nancy (FR); Moussou, Philippe, 54000 Nancy (FR); Enaud, Estelle, 54000 Nancy (FR); Humeau, Catherine, 54210 Ville en Vermois (FR); Girardin, Michel, 54000 Nancy (FR)
(74) Vertreter: Cabinet HERRBURGER

(57) **Zusammenfassung**

Gegenstand der Erfindung sind kosmetische und/oder pharmazeutische Zubereitungen, enthaltend Ester des Retinols und/oder 3,4-Di-Dehydro-Retinols gemäß Formel (I) oder (II) oder (III) oder (IV) 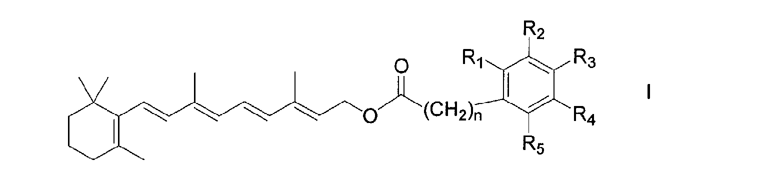 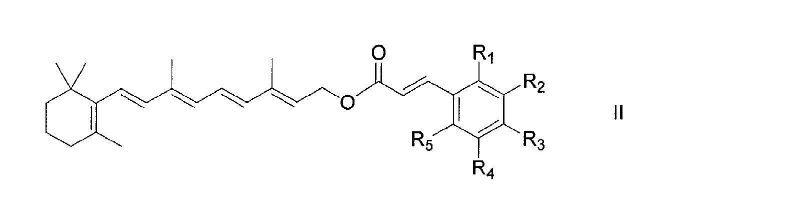 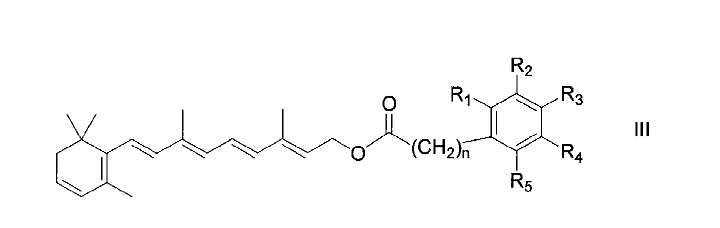 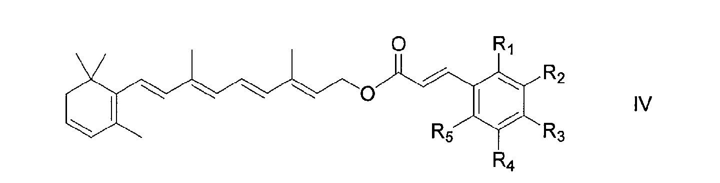 in denen die Reste R1 bis R5 unabhängig voneinander für -H, -OH, -OR', -NHCOR', -NO₂,-SH, -SR', -COOH, -CONHR', -COOR', -CN, -CF₃ stehen und R' einem C₁-C₄ Alkylrest oder Halogenatom entspricht und n ungleich Null ist.

Ein weiterer Gegenstand der Erfindung ist die Herstellung von den Estern des Retinols und/oder 3,4 Di-Dehydro-Retinols vorzugsweise unter enzymatischer Veresterung

## Beschreibung

### Gebiet der Erfindung

Die vorliegende Erfindung bezieht sich auf das Gebiet der Kosmetik und der Dermopharmazie. Sie beschreibt Retinolderivate, deren Herstellung und Verwendung in kosmetischen und/oder pharmazeutischen Zubereitungen.

### Stand der Technik

In den letzten Jahren hat der Einsatz von Retinoiden in kosmetischen Produkten stetig zugenommen. Topische Zubereitungen mit Retinoiden werden mit zahlreichen Indikationen eingesetzt, wie beispielweise zur Behandlung von Akne, zur Vorbeugung und Behandlung von UV-lichtinduzierter Hautalterung, zur Glättung der Haut, zur Verbesserung der Hautelastizität, Hautpigmentierung, oder gegen trockene Haut.
Unter den Retinoiden ist insbesondere Retinol (Vitamin A) hervorzuheben, das eine endogene Komponente menschlicher Haut darstellt. Vitamin A ist bekannt für seine stimulierenden Eigenschaften auf die Epidermisdicke und Kollagensynthese. Es verbessert die Zellerneuerung und Differenzierung, reguliert die Keratinisierung und vermindert die Expression von Matrix-Metallo-Proteinasen der Haut. Im Gegensatz zu der ebenfalls in dermalen Präparaten häufig eingesetzten Retinolsäure ist Vitamin A gut dermal verträglich und führt nicht zu Hautirritationen.
Der Einsatz des Retinols in kosmetischen und pharmazeutischen Formulierungen wird jedoch beschränkt durch seine geringe Stabilität gegenüber Licht, Sauerstoff, oxidierende Stoffe, Metallionen, Temperaturerhöhung Feuchtigkeit und saure pH-Wert-Umgebung. Die Zersetzungsprodukte führen denn zu einer dunklen Verfärbung und einem unangenehmen Geruch. Sie bewirken außerdem ein höhes dermales Irritationspotential. Durch Formulierungstechniken wie Verkapselung, den Einsatz von Antioxidantien oder Veresterung in Form von Palmitat-, Oleat-, Linoleate-, Laurat-, Octanoat-, Butyrat-, Propionat-, und Acetatestern wird versucht, diese Probleme zu beseitigen oder zu mindern. Teilweise sind die Techniken jedoch recht aufwendig und eine zufriedenstellende Lösung ist noch nicht gefunden worden.
Die Europäische Patentanmeldung EP 1057809 schlägt Ester der Kohlensäure mit Retinol zum Einsatz in kosmetischen Formulierungen gegen Akne oder Hautalterung vor. Ebenso zur Behandlung von Hautalterung beschreibt die US 6180670 Retinoidether oder - esterverbindungen mit alpha oder beta-Hydroxy- und Ketosäuren aus der Gruppe der Milchsäure, Zitronensäure, Äpfelsäure, Salicylsäure, Fumarsäure, Oxalsäure, Diphenylglycolsäure, Mandelsäure und Bernsteinsäure.

Das britische Patent GB889266 von 1962 beschreibt Ester von Vitamin A mit substituierten Benzoesäuren, wie m-Nitrobenzoesäure, o- Nitrobenzoesäure, 3,5- Dinitrobenzoesäure, p-Chlorbenzoesäure, p- Methoxybenzoesäure und 3,4,5-Trimethoxybenzoesäure. Nachteil dieser Verbindungen sind jedoch Verunreinigungen, die das Irritationspotential der Ester wiederum erhöhen und eine Umweltbelastung durch Lösungsmittel wie Pyridin, Benzol und THF, die durch die chemische Synthese der Verbindungen bedingt sind.

Die komplexe Aufgabe der vorliegenden Patentanmeldung hat darin bestanden, neue Retinolderivate mit hoher chemischer Stabilität, einem geringen Irritationspotential, guten organoleptischen Eigenschaften und guten Verarbeitungseigenschaften zur Verfügung zu stellen, die sich einfach in hoher Reinheit, mit großer Ausbeute und unter geringer Umweltbelastung herstellen lassen und die Verwendung in kosmetischen und/oder pharmazeutischen Mitteln ermöglichen.

### Beschreibung der Erfindung

Gegenstand der vorliegenden Erfindung sind kosmetische und/oder pharmazeutische Zubereitungen, enthaltend Ester des Retinols und/oder 3,4-Di-Dehydro-Retinols gemäß Formel (1) oder (II) oder (III) oder (IV) 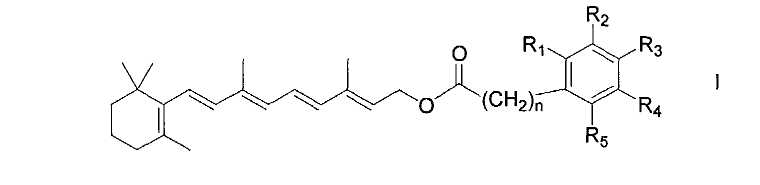 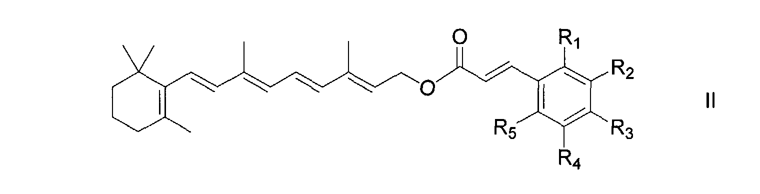 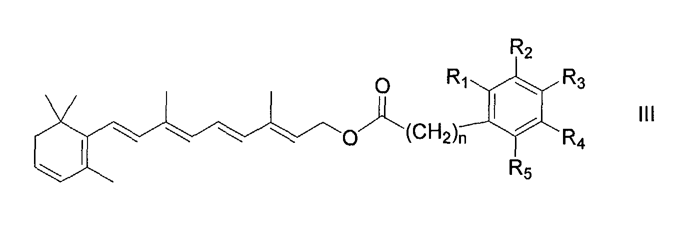 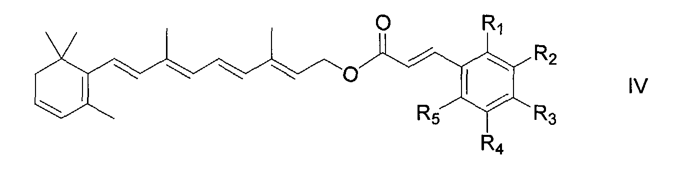 in denen die Reste R1 bis R5 unabhängig voneinander für -H, -OH, -OR', -NHCOR', -NO₂, -SH, -SR', -COOH, -CONHR', -COOR', -CN, -CF₃ stehen und R' einem C₁-C₄ Alkylrest oder Halogenatom entspricht und n ungleich Null ist.

Diese Ester werden hergestellt durch enzymatische Synthese und weisen gegenüber im Stand der Technik bekannten Retinolderivaten eine verbesserte chemische Stabilität, bessere organoleptische Eigenschaften und ein geringes Irritationspotential auf.
Ein weiterer Gegenstand der Erfindung ist ein Verfahren zur Herstellung von diesen Estern des Retinols und/oder 3,4-Di-Dehydro-Retinols gemäß Formel (I) oder (II) oder (III) oder (IV), indem die Verbindungen durch enzymatische Synthese in Form einer direkten Versterung, Interveresterung oder Transveresterung, vorzugsweise mit Lipase hergestellt werden. Es werden insbesondere Lipasen eingesetzt aus Mikroorganismen ausgewählt aus der Gruppe, die gebildet wird von Candida antarctica, Rhizomucor miehei, Candida cylindracea und Rhizopus arrhizus.

Überraschender Weise wurde festgestellt, dass die Veresterung von Vitamin A mit Substanzen die gegen freie Radikale, als Antioxidans und zum UV-Schutz wirksam sind, nach topischer Applikation eine synergistische Wirkung gegenüber den Einzelkomponenten zeigt. Bedingt durch den langsamen enzymatischen Abbau der Retinol- und 3,4-Di-Dehydro-Retinolester durch nichtspezifische hydrolytische Enzyme nach der Absorption im Hautgewebe kommt es zu einer langanhaltenden Schutzwirkung. Nach Esterspaltung können das freie Retinol und die freien Antiradikal-, Antioxidans- und Anti-UV-wirksubstanzen ihre Wirkung in der Haut entfalten. Die chemisch sehr stabilen bifunktionellen Moleküle zeigen eine weitaus verbesserte biologische Effektivität als die Summe der Einzelkomponenten. Das enzymatisch durchgeführte Verfahren zur Herstellung der Ester hat gegenüber herkömmlichen chemischen Verfahren den Vorteil, dass es zu Endprodukten mit hoher Reinheit führt, die sich durch eine gute Toleranz nach topischer Applikation auszeichnen. Die Vermeidung toxischer Lösungsmittel wie beispielsweise Pyridin, Benzol, Chloroform und THF, von Salzen und Verunreinigungsprodukten durch Retinolabbau führt beim enzymatischen Verfahren unter milden Bedingungen dazu, dass aufwendige Aufreiningungsschritte nicht notwendig sind. Das enzymatisch hergestellte Produkt zeichnet sich durch ein sehr geringes Irritationspotential aus und weist eine hohe chemische Stabilität auf, so dass kosmetische und/oder pharmazeutische Zubereitungen, die diese Ester enthalten, neben der guten physiko-chemischen Stabilität auch eine hervorragende Verträglichkeit aufweisen.

Je nach dem mit Retinol oder 3,4-Di-Dehydro-Retinol veresterten Substituenten können die bifunktionalen Moleküle zur Herstellung unterschiedlicher kosmetischer und/oder pharmazeutischer Zubereitungen eingesetzt werden. In Form eines Antioxidans und bei Kopplung mit Anti-Radikal-Agentien erfolgt ihr Einsatz in Mitteln gegen Hautalterung, Hautschädigung oder gegen entzündliche Prozesse wie beispielsweise Akne sowie zur Beschleunigung der Zellerneuerung und Zelldifferenzierung.
Die Veresterung mit UV-Filtern bietet sich zum Einsatz in Sonnenschutzmitteln an. Derartige Derivate dienen auch der Vorbeugung von Folgeerkrankungen, da sie zum Schutz der zellulären Adenosindinukleotide (ADN) vor UV-Licht Schädigungen eingesetzt werden können.

Dabei kann bedingt durch die Bifunktionalität der Ester ein komplexer Ablauf wie die Hautalterung oder Zellschädigung über unterschiedliche Angriffsmechanismen positiv beeinflußt werden. Obgleich die Moleküle durch die ubiquitären Enzyme nach Absorption in ihre Einzelkomponenten gespalten werden, weisen sie eine synergistische Wirkung auf.
Insbesondere in Zubereitungen gegen trockene und rauhe Haut oder gegen entzündliche Prozesse wirkt sich die durch die kontinuierliche enzymatische Hydrolyse langanhaltende Verfügbarkeit der Aktivsubstanzen positiv aus.

Wirkungsweisen, die für Vitamin A - derivate beschrieben werden, wie die Induktion des epidermalen Zellwachstums, Regulierung der Keratinisierung, Steigerung der Collagensynthese und Verminderung der Expression von Matrix-Metallo-Proteinase können ebenfalls durch den Metabolismus der erfindungsgemäßen Ester über einen langen Zeitraum aufrechterhalten werden.

### Ester des Retinols und/oder 3,4-Di-Dehydro-Retinols

Die Ester des Retinols und/oder 3,4-Di-Dehydro-Retinols gemäß Formel (I) oder (II) oder (III) oder (IV), in denen die Reste R1 bis R5 unabhängig voneinander für -H, -OH, -OR', -NHCOR', -NO₂, -SH, -SR', -COOH, -CONHR', -COOR', -CN, -CF₃ stehen und R' einem C₁-C₄ Alkylrest oder Halogenatom entspricht und n ungleich Null ist, die vorzugsweise in den kosmetischen und/oder pharmazeutischen Zubereitungen eingesetzt werden, sind am Phenylring mit Resten wie -H und/oder-OH und/oder -OMe substituiert, und haben als n 1 bis 3 CH₂-Gruppen.

Die Herstellung der Retinol- und/oder 3,4-Di-Dehydro-Retinolester erfolgt durch enzymatische Synthese. Als Enzyme werden bevorzugt Lipasen eingesetzt. Die Lipasen stammen aus Mikroorganismen, die ausgewählt sind aus der Gruppe, die gebildet wird von Candida antarctica, Rhizomucor miehei, Candida cylindracea und Rhizopus arrhizus.
Es werden Methoden wie die direkte Veresterung, Interveresterung oder Transveresterung angewendet, wobei ein Acyldonor als Lösungsmittel oder Lösungsmittel ausgewählt aus der Gruppe, die gebildet wird von Hexane, Heptane, tert-Butanol, 2-Methyl-2-Butanol, 4-Hydroxy-4-Methyl-2-Pentanon zur Lösung des Substrates verwendet werden. Um das Reaktionsgleichgewicht auf die Seite der Retinolester zu verschieben, werden je nach angewendeter Reaktion - direkte, Inter- oder Transveresterung - Wasser, Alkohol oder Ester durch Vakuum, Molekularsieb, Evaporation oder andere Techniken nach der Umsetzung aus der Reaktion entfernt. Substrate und Produkte können vor Oxidation geschützt werden, indem man die Reaktion unter Lichtausschluß und/oder in Inertgasathmosphäre ablaufen läßt.
Die eingesetzte Lipase - in ihrer freien Form, auf einem inerten Träger oder immobilisiert - kann nach der Reaktion recycelt werden. Die Substrate werden in equimolaren Mengen oder mit einem Überschuß an Acyldonor eingesetzt und die Reaktion kann in diskontinuierlicher Weise oder semi-kontinuierlich durch Zugabe von Retinol resp. Retinolester bei der Transveresterung stattfinden.

### Gewerbliche Anwendung

Die erfindungsgemäßen kosmetischen und/oder pharmazeutischen Zubereitungen enthalten 0,0001 bis 10 Gew.%, vorzugsweise 0,01 bis 8 Gew.% und besonders bevorzugt 0,1 bis 5 Gew.% der Retinol- und/oder 3,4-Di-Dehydro-Retinolester.

Die durch Formel (1) bis (IV) beschriebenen Ester können zur Herstellung kosmetischer und/oder pharmazeutischer Mittel, wie beispielsweise Haarshampoos, Haarlotionen, Schaumbäder, Duschbäder, Cremes, Gele, Lotionen, alkoholische und wäßrig/alkoholische Lösungen, Emulsionen, Wachs/ Fett-Massen, Stiftpräparaten, Pudern oder Salben, vornehmlich jedoch Erzeugnisse für Gesicht und Körper, Tag- oder Nachtkosmetika, Sonnenschutzmittel, kräftigende, regenerierende Erzeugnisse, Anti-Faltenkosmetika, Schlankheitskurenmittel und Mittel gegen Alterungsprozesse dienen.
Diese Mittel können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, weitere Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Monound/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
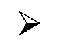 Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
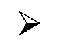 Alkyl- und/oder Alkenyloligoglykoside mit 8 bis 22 Kohlenstoffatomen im Alk(en)ylrest und deren ethoxylierte Analoga;
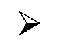 Anlagerungsprodukte von 1 bis 15 Mol Ethylenoxid an Ricinusöl und/oder gehärtetes Ricinusöl;
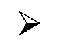 Anlagerungsprodukte von 15 bis 60 Mol Ethylenoxid an Ricinusöl und/oder gehärtetes Ricinusöl;
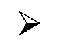 Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
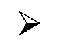 Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
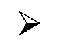 Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
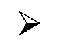 Mono-, Di- und Trialkylphosphate sowie Mono-, Di- und/oder Tri-PEG-alkylphosphate und deren Salze;
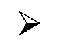 Wollwachsalkohole;
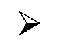 Polysiloxan-Polyalkyl-Polyether-Copolymere bzw. entsprechende Derivate;
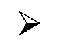 Block-Copolymere z.B. Polyethylenglycol-30 Dipolyhydroxystearate;
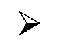 Polymeremulgatoren, z.B. Pemulen-Typen (TR-1 ,TR-2) von Goodrich;
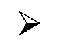 Polyalkylenglycole sowie
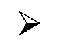 Glycerincarbonat.

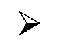 Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

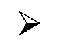 Alkyl- und/oder Alkenvioligogivkoside

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

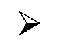 Partialglyceride

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

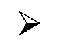 Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

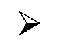 Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® Gl 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

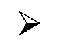 Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

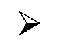 Amphothere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyloder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropion-säuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminiumund/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide, sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/lsobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
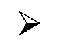 3-Benzylidencampher bzw. 3-Benzylidennorcampher und dessen Derivate, z.B. 3-(4-Methylbenzyliden)campher;
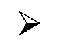 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
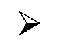 Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
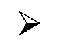 Ester der Salicylsäure, vorzugsweise Salicylsäure-2-ethylhexylester, Salicylsäure-4-isopropylbenzylester, Salicylsäurehomomenthylester;
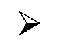 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
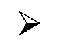 Ester der Benzalmalonsäure, vorzugsweise 4-Methoxybenzmalonsäuredi-2-ethylhexyl-ester;
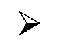 Triazinderivate, wie z.B. 2,4,6-Trianilino-(p-carbo-2'-ethyl-1'-hexyloxy)-1,3,5-triazin und Octyl Triazon, oder Dioctyl Butamido Triazone (Uvasorb® HEB);
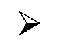 Propan-1,3-dione, wie z.B. 1-(4-tert.Butylphenyl)-3-(4'methoxyphenyl)propan-1,3-dion;
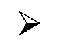 Ketotricyclo(521.0)decan-Derivate.

Als wasserlösliche Substanzen kommen in Frage:
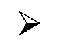 2-Phenylbenzimidazol-5-sulfonsäure und deren Alkali-, Erdalkali-, Ammonium-, Alkylammonium-, Alkanolammonium- und Glucammoniumsalze;
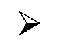 Sulfonsäurederivate von Benzophenonen, vorzugsweise 2-Hydroxy-4-methoxybenzophenon-5-sulfonsäure und ihre Salze;
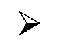 Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert. Butylphenyl)-3-(4'-methoxyphenyl)propan-1,3-dion, 4-tert-Butyl-4`methoxydibenzoyimethan (Parsol® 1789), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans" z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt.

Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. y-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

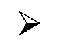 Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxy-diphenylether (Tridosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

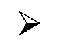 Enzyminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

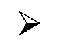 Geruchsabsorber

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

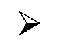 Antitranspirantien

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
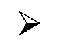 adstringierende Wirkstoffe,
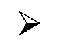 Ölkomponenten,
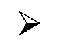 nichtionische Emulgatoren,
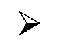 Coemulgatoren,
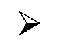 Konsistenzgeber,
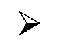 Hilfsstoffe wie z. B. Verdicker oder Komplexierungsmittel und/oder
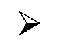 nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirko-nium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
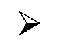 entzündungshemmende, hautschützende oder wohlriechende ätherische Öle,
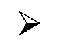 synthetische hautschützende Wirkstoffe und/oder
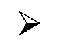 öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-(-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wäßrige Phasen können Montmorillonite, Clay Mineralstoffe, Pemulen sowie alkylmodifizierte Carbopoltypen (Goodrich) dienen.

### Insekten-Repellentien

Als Insekten-Repellentien kommen N,N-Diethyl-m-toluamid, 1,2-Pentandiol oder Ethyl Butylacetylaminopropionate in Frage

### Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
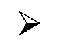 Glycerin;
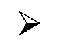 Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
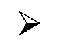 technische Oligoglyceringemische mit einem Eigenkondensationsgrad von 1,5 bis 10 wie etwa technische Diglyceringemische mit einem Diglyceringehalt von 40 bis 50 Gew.-%;
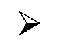 Methyolverbindungen, wie insbesondere Trimethylolethan, Trimethylolpropan, Trimethylolbutan, Pentaerythrit und Dipentaerythrit;
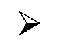 Niedrigalkylglucoside, insbesondere solche mit 1 bis 8 Kohlenstoffen im Alkylrest, wie beispielsweise Methyl- und Butylglucosid;
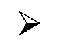 Zuckeralkohole mit 5 bis 12 Kohlenstoffatomen, wie beispielsweise Sorbit oder Mannit,
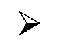 Zucker mit 5 bis 12 Kohlenstoffatomen, wie beispielsweise Glucose oder Saccharose;
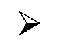 Aminozucker, wie beispielsweise Glucamin;
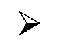 Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, a-lsomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, a-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden. Beispiele sind Kochenillerot A (C.1. 16255), Patentblau V (C.1.42051), Indigotin (C.1.73015), Chlorophyllin (C.l.75810 Chinolingelb (C.1.47005), Titandioxid (C.1.77891) Indanthrenblau RS (C.1. 69800) und Krapplack (C.1.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

### Beispiele

### Herstellung von Retinolzimtsäureester

### Beispiel 1 - Transveresterung

2 ml Ethylcinnamat (11,9 mmol), 50 mg Retinol (0,175 mmol), 200 mg Butylhydroxytoluol (BHT) als Antioxidans und 100 mg immobilisierte Lipase von *Candida antarctica* Novozym 435® wurden unter Rühren und Lichtausschluß in einem Reaktor zusammengefügt. Die Reaktion lief bei 50° C und 200 mbar Vakuum ab. Nach 7 Stunden war die Umsetzung von Retinol zu Retinolcinnamat vollständig erfolgt, der Endpunkt wurde durch eine HPLC-analyse ermittelt.

### Beispiel 2 - Interveresterung

2 ml Ethylcinnamat (11,9 mmol), 100 mg Retinolacetat (0,3 mmol), 200 mg Butylhydroxytoluol (BHT) als Antioxidans und 100 mg immobilisierte Lipase von *Candida antarctica* Novozym 435® wurden unter Rühren und Lichtausschluß in einem Reaktor zusammengefügt. Die Reaktion lief bei 50° C und 200 mbar Vakuum ab. Nach 28 Stunden war die Umsetzung von Retinolacetat zu Retinolcinnamat vollständig erfolgt, der Endpunkt wurde durch eine HPLC-analyse ermittelt.

### Beispiel 3 - Direkte Veresterung

2 ml 2-Methyl-2-butanol als Lösungsmittel, 1g Zimtsäure (6,7 mmol), 50 mg Retinol (0,175 mmol), 200 mg Butylhydroxytoluol (BHT) als Antioxidans und 100 mg immobilisierte Lipase von *Candida antarctica* Novozym 435® wurden unter Rühren und Lichtausschluß in einem Reaktor zusammengefügt. Die Reaktion lief bei 50° C und 200 mbar Vakuum ab. Nach 4 Stunden war die Umsetzung von Retinol zu Retinolcinnamat vollständig erfolgt, der Endpunkt wurde durch eine HPLC-analyse ermittelt.

In den Tabellen 1a und 1b sind weitere Vorschläge für Rezepturen mit Retinolcinnamat gemäß Beispiel 1 und 3 (Laboratoires Sérobiologiques) beschrieben.

**Tabelle 1a**

| **Kosmetische Zubereitungen (Mengenangaben in Gew.-%)** | | | |
|---|---|---|---|
| **Zusammensetzung (INCI)** | **1** | **2** | **3** |
| **Emulgade® SE** Glyceryl Sterate (and) Ceteareth 12/20 (and) Cetearyl Alcohol (and) Cetyl Palmitate | 5,0 | 5,0 | - |
| **Lameform® TGI** Polyglyceryl-3 Isostearate | - | - | 4,0 |
| **Monomuls® 90-018** Glyceryl Oleate | - | - | 2,0 |
| **Cetiol® OE** Dicaprylyl Ether | - | - | 5,0 |
| **Cetiol® PGL** Hexyldecanol (and) Hexyldecyl Laurate | - | - | 10,0 |
| **Cetiol® SN** Cetearyl Isononanoate | 3,0 | 3,0 | - |
| **Cetiol® V** Decyl Oleate | 3,0 | 3,0 | - |
| **Myritol® 318** Coco Caprylate Caprate | - | - | 5,0 |
| **Bees Wax** | - | - | 7,0 |
| **Nutrilan® Keratin W** Hydrolyzed Keratin | 40,0 | 60,0 | - |
| **Gluadin® WK** Sodium Cocoyl Hydrolyzed Wheat Protein | - | - | 5,0 |
| **Magnesium Sulfate Hepta Hydrate** | - | - | 1,0 |
| **Glycerin** (86 Gew.-%ig) | 3,0 | 3,0 | 5,0 |
| **Retinolcinnamat gemäß Beispiel 1** | 2,0 | 4,0 | 2,0 |
| **Aqua conservata** | ad 100,0 | ad 100,0 | ad 100,0 |
| (1) Softcreme, (2) Feuchtigkeitsemulsion, (3) Nachtcreme | | | |

**Tabelle 1b**

| **Kosmetische Zubereitungen (Mengenangaben in Gew.-%)** | | | | |
|---|---|---|---|---|
| **Zusammensetzung (INCI)** | **4** | **5** | **6** | **7** |
| **Dehymuls® PGPH** Polyglyceryl-2 Dipolyhydroxystearate | 2,0 | 3,0 | - | - |
| **Lameform® TGI** Polyglyceryl-3 Diisostearate | 4,0 | 1,0 | - | - |
| **Eumulgin VL 75** Polyglyceryl-2 Dipolyhydroxystearate (and) Lauryl Glucoside (and) Glycerin | - | - | 3,5 | - |
| **Bees Wax** | 3,0 | 2,0 | - | - |
| **Cutina® GMS** Glyceryl Stearate | - | - | 2,0 | 4,0 |
| **Lanette® O** Cetearyl Alcohol | - | - | 4,0 | 1,0 |
| **Antaron® V 216** PVP / Hexadecene Copolymer | - | - | 3,0 | 2,0 |
| **Plantaren® 818** Cocoglycerides | 5,0 | - | 6,0 | 5,0 |
| **Finsolv® TN** C12/15 Alkyl Benzoate | - | 6,0 | - | 2,0 |
| **Dioctyl Carbonate** | 5,0 | 4,0 | 5,0 | 6,0 |
| **Cetiol® J 600** Oleyl Erucate | 2,0 | - | 3,0 | 4,0 |
| **Cetiol® OE** Dicaprylyl Ether | 3,0 | - | 1,0 | - |
| **Mineral Oil** | - | 4,0 | 2,0 | - |
| **Cetiol® PGL** Hexadecanol (and) Hexyldecyl Laurate | - | 7,0 | - | - |
| **Panthenol / Bisabolol** | 1,2 | 1,2 | 1,2 | 1,2 |
| **Copherol® F 1300** Tocopherol / Tocopheyl Acetate | 0,5 | 1,0 | 1,0 | 2,0 |
| **Neo Heliopan® Hydro** Sodium Phenylbenzimidazole Sulfonate | 2,0 | - | - | - |
| **Neo Heliopan® 303** Octocrylrin | - | 3,0 | 2,0 | 5,0 |
| **Neo Heliopan® BB** Benzophenone-3 | 0,5 | - | - | - |
| **Uvinul® T 150** Octyl triazone | 2,0 | 2,0 | 1,0 | 2,0 |
| **Zinc Oxide** | - | 6,0 | - | 5,0 |
| **Titanium Dioxide** | - | 2,0 | - | - |
| **Glycerin (86 Gew.-%ig)** | 5,0 | 5,0 | 5,0 | 5,0 |
| **Retinolcinnamat gemäß Beispiel 3** | 1,0 | 2,0 | 5,0 | 4,0 |
| **Aqua conservata** | ad 100,0 | ad 100,0 | ad 100,0 | ad 100,0 |
| (4) W/O-Sonnenschutzcreme, (5) W/O-Sonnenschutzlotion, (6) O/W-Sonnenschutzcreme (7) O/W-Sonnenschutzlotion | | | | |

## Patentansprüche

1. Kosmetische und/oder pharmazeutische Zubereitungen, enthaltend Ester des Retinols und/oder 3,4-Di-Dehydro-Retinols gemäß Formel (1) oder (II) oder (III) oder (IV) 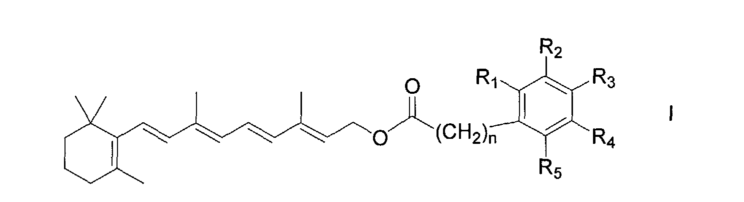 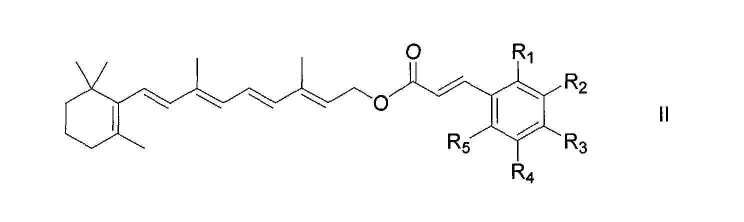 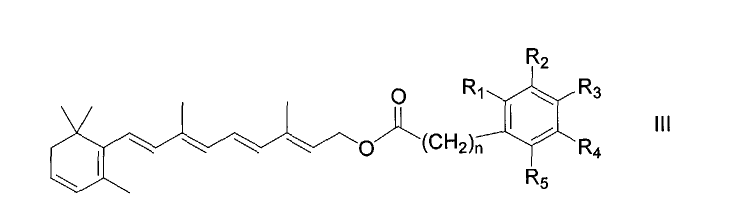 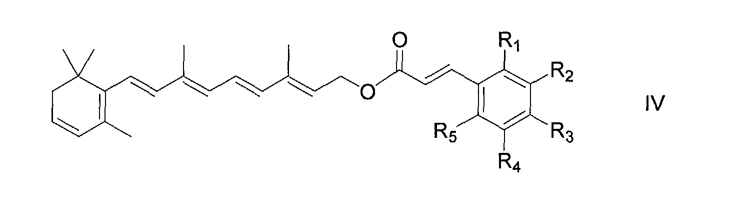 in denen die Reste R1 bis R5 unabhängig voneinander für -H, -OH, -OR', -NHCOR', -NO₂, -SH, -SR', -COOH, -CONHR', -COOR', -CN, -CF₃ stehen und R' einem C₁-C₄ Alkylrest oder Halogenatom entspricht und n ungleich Null ist.

2. Kosmetische und/oder pharmazeutische Zubereitungen nach Anspruch 1, charakterisiert dadurch, dass die Ester gemäß Formel 1 oder II in Mengen von 0,0001 bis 10 Gew. % - bezogen auf die Formulierung eingesetzt werden.

3. Verfahren zur Herstellung von Estern des Retinols und/oder 3,4-Di-Dehydro-Retinols gemäß Formel (I) oder (II) oder (III) oder (IV)
in denen die Reste R1 bis R5 unabhängig voneinander für -H, -OH, -OR', -NHCOR', -NO₂, -SH, -SR', -COOH, -CONHR', -COOR', -CN, -CF₃ stehen und R' einem C₁-C₄ Alkylrest oder Halogenatom entspricht und n ungleich Null ist., indem man eine enzymatische Synthese in Form einer direkten Veresterung, Interveresterung oder Transveresterung durchführt.

4. Herstellung gemäß Anspruch 3 dadurch charakterisiert, dass als Enzym Lipase verwendet wird.

5. Herstellung gemäß Anspruch 3 oder 4, dadurch charakterisiert, dass als Enzym Lipasen verwendet werden, die aus Candida antarctica, Rhizomucor miehei, Candida cylindracea und Rhizopus arrhizus erhalten werden.

6. Verwendung von Estern des Retinols und/oder 3,4-Di-Dehydro-Retinols gemäß Formel (1) oder (II) oder (III) oder (IV), in denen die Reste R1 bis R5 unabhängig voneinander für -H, -OH, -OR', -NHCOR', -NÖ₂, -SH, -SR', -COOH, -CONHR', -COOR', -CN, -CF₃ stehen und R' einem C₁-C₄ Alkylrest oder Halogenatom entspricht und n ungleich Null ist, zur Herstellung von kosmetischen und/oder pharmazeutischen Zubereitungen gegen Hautalterung.

7. Verwendung von Estern des Retinols und/oder 3,4-Di-Dehydro-Retinols gemäß Formel (1) oder (II) oder (III) oder (IV), in denen die Reste R1 bis R5 unabhängig voneinander für -H, -OH, -OR', -NHCOR', -NO₂, -SH, -SR', -COOH, -CONHR', -COOR', -CN, -CF₃ stehen und R' einem C₁-C₄ Alkylrest oder Halogenatom entspricht und n ungleich Null ist, zur Herstellung von kosmetischen und/oder pharmazeutischen Zubereitungen zur Induktion des epidermalen Zellwachstums, zur Regulierung der Keratinisierung, zur Steigerung der Collagensynthese und zur Verminderung der Expression von Matrix-Metallo-Proteinase.

8. Verwendung von Estern des Retinols und/oder 3,4-Di-Dehydro-Retinols gemäß Formel (1) oder (II) oder (III) oder (IV), in denen die Reste R1 bis R5 unabhängig voneinander für -H, -OH, -OR', -NHCOR', -NO₂, -SH, -SR', -COOH, -CONHR', -COOR', -CN, -CF₃ stehen und R' einem C₁-C₄ Alkylrest oder Halogenatom entspricht und n ungleich Null ist zur Herstellung von Sonnenschutzmitteln.

9. Verwendung von Estern des Retinols und/oder 3,4-Di-Dehydro-Retinols gemäß Formel (1) oder (II) oder (III) oder (IV), in denen die Reste R1 bis R5 unabhängig voneinander für -H, -OH, -OR', -NHCOR', -NO₂, -SH, -SR', -COOH, -CONHR', -COOR', -CN, -CF₃ stehen und R' einem C₁-C₄ Alkylrest oder Halogenatom entspricht und n ungleich Null ist, zur Herstellung von kosmetischen und/oder pharmazeutischen Zubereitungen zum Schutz der zellulären ADN vor UV-Licht Schädigungen.

10. Verwendung von Estern des Retinols und/oder 3,4-Di-Dehydro-Retinols gemäß Formel (1) oder (II) oder (III) oder (IV), in denen die Reste R1 bis R5 unabhängig voneinander für -H, -OH, -OR', -NHCOR', -NO₂, -SH, -SR', -COOH, -CONHR', -COOR', -CN, -CF₃ stehen und R' einem C₁-C₄ Alkylrest oder Halogenatom entspricht und n ungleich Null ist zur Herstellung von kosmetischen und/oder pharmazeutischen Zubereitungen gegen trockene und/oder rauhe Haut.

11. Verwendung von Estern des Retinols und/oder 3,4-Di-Dehydro-Retinols gemäß Formel (I) oder (II) oder (III) oder (IV), in denen die Reste R1 bis R5 unabhängig voneinander für -H, -OH, -OR', -NHCOR', -NO -SH, -SR', -COOH, -CONHR', -COOR', -CN, -CF3 stehen und R' einem C1-C4 Alkylrest oder Halogenatom entspricht und n ungleich Null ist zur Herstellung von kosmetischen und/oder pharmazeutischen Zubereitungen zur Beschleunigung der Zellerneuerung und Zelldifferenzierung.

12. Verwendung von Estern des Retinols und/oder 3,4-Di-Dehydro-Retinols gemäß Formel (1) oder (II) oder (III) oder (IV), in denen die Reste R1 bis R5 unabhängig voneinander für -H, -OH, -OR', -NHCOR', -NO₂, -SH, -SR', -COOH, -CONHR', -COOR', -CN, -CF₃ stehen und R' einem C₁-C₄ Alkylrest oder Halogenatom entspricht und n ungleich Null ist zur Herstellung von Anti-Aknemitteln.
